# EUROPEAN PATENT SPECIFICATION

(11) **EP 4 340 746 B1**
(45) Date of publication and mention of the grant of the patent: **28.10.2026**
(21) Application number: 22726182.3
(22) Date of filing: 13.05.2022
(51) Int. Cl.: A61B 17/072, A61B 17/00

(54) **SURGICAL STAPLING DEVICE WITH RELOAD ASSEMBLY REMOVAL LOCKOUT**
CHIRURGISCHE KLAMMERVORRICHTUNG MIT WIEDERLADEANORDNUNGSENTFERNUNGSSPERRE
DISPOSITIF D'AGRAFAGE CHIRURGICAL DOTÉ D'UN MÉCANISME DE VERROUILLAGE EMPÊCHANT LE RETRAIT D'UN ENSEMBLE DE RECHARGEMENT

(30) Priority: 19.05.2021 US 202117324440
(43) Date of publication of application: 27.03.2024
(73) Proprietor: Covidien LP, Mansfield, MA 02048 (US)
(72) Inventor: WILLIAMS, Justin P., North Haven, CT 06473 (US)
(74) Representative: Zimmermann & Partner Patentanwälte mbB
(86) International application number: PCT/IB2022/054489
(87) International publication number: WO 2022/243826

(56) References cited:
- US-A1- 2009 206 137
- US-A1- 2011 163 148
- US-A1- 2016 174 976

## Description

### FIELD

This technology is generally related to surgical stapling devices and, more particularly, to surgical stapling devices that include replaceable reload assemblies.

### BACKGROUND

Surgical stapling devices for simultaneously cutting and stapling tissue are known in the art and are commonly used during surgical procedures to reduce the time required to suture tissue. Such devices are available in endoscopic and open configurations. Endoscopic surgical stapling devices reduce trauma inflicted on a patient during a surgical procedure to minimize patient discomfort and reduce patient recovery times.

Endoscopic surgical stapling devices include an adapter assembly that supports a tool assembly and defines a longitudinal axis. Typically, the tool assembly is coupled to a distal portion of the adapter assembly by a pivot member to facilitate articulation of the tool assembly about an axis transverse to the longitudinal axis of the adapter assembly. Articulation of the tool assembly improves access to tissue within a body cavity during a surgical procedure.

Some surgical stapling devices include a tool assembly that forms part of a replaceable reload assembly that is releasably coupled to the adapter assembly. During a surgical procedure, a spent reload assembly can be removed and replaced on the surgical stapling device to facilitate reuse of the surgical stapling device. Typically, the reload assembly is coupled to the adapter assembly with a bayonet type coupling. During replacement, a proximal portion of the reload assembly is inserted into a distal portion of the adapter assembly and rotated to lock the reload assembly to the body, and to couple the drive and articulation components of the adapter assembly to the drive and articulation components of the reload assembly. To ensure that the drive and articulation components of the adapter assembly and the reload assembly are properly coupled during attachment of the reload assembly to the adapter assembly, it is important that the drive component of the adapter assembly is in a fully retracted position and that the articulation component of the adapter assembly is in a non-articulated position.
US2011/163148A1 describes a surgical stapling device having an articulatable tool member for applying linear rows of staples to tissue and concurrently incising the tissue between the linear rows of staples. US2016/174976A1 describes a surgical instrument. In one form, the surgical instrument includes a housing that has an interchangeable shaft assembly removably attached thereto. The interchangeable shaft assembly includes an elongate shaft that has a surgical end effector operably coupled thereto for selective articulation relative to the elongate shaft. An articulation system is configured to selectively apply articulation motions to the surgical end effector when the articulation system is in an actuated orientation. A locking arrangement prevents detachment of the interchangeable shaft assembly from the housing when the articulation system is in the actuated orientation.

### SUMMARY

The invention is defined by the appended independent claim. Optional features are set out in the appended dependent claims.

This disclosure generally relates to stapling devices with replaceable reload assemblies that include a reload release mechanism that is movable from a locked position in which the reload assembly is locked onto an adapter assembly to an unlocked position in which the reload assembly can be removed from the adapter assembly. The adapter assembly includes a lockout that prevents movement of the reload release mechanism from the locked position to the unlocked position unless the stapling device is in a non-articulated, unfired position.

One aspect of the disclosure is directed to an adapter assembly that includes a firing rod, an articulation assembly, a release mechanism, and a lockout. The firing rod includes a proximal portion and a distal portion and is movable between retracted and advanced positions. The articulation assembly includes an articulation lever, a coupling member, and an articulation link. The coupling member is positioned between the articulation lever and the articulation link. The articulation lever is rotatable to move the coupling member longitudinally between a neutral position and non-neutral positions to move the articulation link between advanced and retracted positions. The release mechanism is movable from a locked position to an unlocked position. The lockout is movable from an unlocked position to a locked position in response to movement of the coupling member from the neutral position to one of the non-neutral positions and/or in response to movement of the firing rod from the retracted position towards the advanced position. The lockout is configured to prevent movement of the release mechanism from its locked position to its unlocked position when the lockout is in its locked position.

The lockout includes a body having a first protrusion and a second protrusion, the coupling member of the articulation assembly defines a first notch, and the firing rod defines a second notch.

The first protrusion is positioned within the first notch when the coupling member is in the neutral position, and the second protrusion is positioned within the second notch when the firing rod is in its retracted position.

In certain aspects of the disclosure, the body of the lockout includes a stop member, and the release mechanism includes a release button that defines a cavity.

In aspects of the disclosure, the stop member of the body of the lockout is received within the cavity when the lockout is in its locked position.

In some aspects of the disclosure, the lockout includes a biasing member that urges the lockout towards its unlocked position.

In certain aspects of the disclosure, the biasing member includes a leaf spring.

In aspects of the disclosure, the body of the lockout is supported in cantilevered fashion from the leaf spring.

In some aspects of the disclosure, the body includes a cylindrical pivot member, and the lockout is pivotable on the cylindrical pivot member between its locked and unlocked positions.

In certain aspects of the disclosure, the release mechanism includes a release member that is coupled to the release button.

Other aspects of the disclosure are directed to an adapter assembly that includes an articulation assembly, a release mechanism, and a lockout. The articulation assembly includes an articulation lever, a coupling member, and an articulation link. The coupling member is positioned between the articulation lever and the articulation link. The articulation lever is rotatable to move the coupling member longitudinally between a neutral position and non-neutral positions to move the articulation link between advanced and retracted positions. The release mechanism is movable from a locked position to an unlocked position. The lockout is movable from an unlocked position to a locked position in response to movement of the coupling member from the neutral position to one of the non-neutral positions. The lockout is configured to prevent movement of the release mechanism from its locked position to its unlocked position when the lockout is in its locked position.

Other aspects of the disclosure are directed to a surgical stapling device that includes a reload assembly and an adapter assembly. The reload assembly includes a proximal body and a tool assembly. The proximal body defines a first longitudinal axis. The tool assembly is pivotably coupled to the proximal body by a pivot member that defines an articulation axis that is transverse to the first longitudinal axis. The tool assembly includes a cartridge assembly and an anvil. The proximal body includes a drive assembly and a first articulation link that has a proximal portion and a distal portion. The distal portion of the first articulation link is coupled to the tool assembly such that movement of the first articulation link causes articulation of the tool assembly about the articulation axis between a non-articulated position and articulated positions. The drive assembly is movable between retracted and advanced positions to actuate the tool assembly. The adapter assembly defines a second longitudinal axis and has a distal portion that is releasably coupled to the reload assembly. The adapter assembly includes a firing rod, an articulation assembly, a release mechanism, and a lockout. The firing rod has a proximal portion and a distal portion. The distal portion of the firing rod is adapted to be coupled to the drive assembly of the reload assembly when the reload assembly is coupled to the adapter assembly. The firing rod is movable between retracted and advanced positions to move the drive assembly of the reload assembly between its retracted and advanced positions to actuate the tool assembly. The articulation assembly includes an articulation lever, a coupling member, and a second articulation link. The coupling member is positioned between the articulation lever and the first articulation link. The articulation lever is rotatable to move the coupling member longitudinally between a neutral position and non-neutral positions to move the first articulation link between advanced and retracted positions. The second articulation link is adapted to be coupled to the first articulation link when the reload assembly is coupled to the adapter assembly such that longitudinal movement of the second articulation link causes longitudinal movement of the first articulation link to articulate the tool assembly about the articulation axis. The release mechanism is movable from a locked position to an unlocked position. In the locked position, the release mechanism prevents the reload assembly from being uncoupled from the adapter assembly. The lockout is movable from an unlocked position to a locked position in response to movement of the coupling member from the neutral position to one of the non-neutral positions and/or in response to movement of the firing rod from its retracted position towards its advanced position. The lockout is configured to prevent movement of the release mechanism from its locked position to its unlocked position when the lockout is in its locked position.

In aspects of the disclosure, a handle assembly is coupled to the adapter assembly.

In some aspects of the disclosure, the surgical stapling device includes a rotation knob that is rotatably coupled to the handle assembly and secured to the proximal portion of the adapter assembly such that rotation of the rotation knob in relation to the handle assembly rotates the adapter assembly and the tool assembly about the second longitudinal axis.

Other features of the disclosure will be appreciated from the following description.

### BRIEF DESCRIPTION OF DRAWINGS

Various aspects of the disclosure are described herein below with reference to the drawings, wherein:
FIG. 1 is a side perspective view of a surgical stapling device according to aspects of the disclosure;
FIG. 2 is a side perspective view of the surgical stapling device of FIG. 1 with a reload assembly separated from an adapter assembly of the stapling device;
FIG. 3 is a enlarged view of the indicated area of detail shown in FIG. 2;
FIG. 4 is a side perspective view of the adapter assembly of the stapling device shown in FIG. 1 with an outer tube and rotation knob of the adapter assembly shown in phantom showing a reload release assembly;
FIG. 5 is a side perspective view of an interface between the reload assembly and the adapter assembly with the reload release assembly in a locked position;
FIG. 6 is a side perspective view of the interface between the reload assembly and the adapter assembly shown in FIG. 5 with the reload release assembly in an unlocked position;
FIG. 7 is a side perspective view of the interface between the reload assembly and the adapter assembly shown in FIG. 6 with the reload release assembly rotated to a release position;
FIG. 8 is a side perspective view of the interface between the reload assembly and the adapter assembly shown in FIG. 6 with the reload release assembly separated from the adapter assembly;
FIG. 9 is an exploded perspective view of the lockout, a reload release mechanism, an articulation assembly, and drive member of the central portion of the surgical stapling device shown in FIG. 3;
FIG. 10 is a side perspective view of a first side of a central portion of the surgical stapling device shown in FIG. 1 with a rotation knob shown in phantom, an articulation yoke in a neutral position, and a lockout in an unlocked position;
FIG. 11 is a bottom perspective view of the lockout shown in FIG. 9;
FIG. 12 is a side perspective view of the articulation yoke shown in FIG. 9;
FIG. 13 is a side perspective view from the first side of the central portion of the surgical stapling device shown in FIG. 10 with a reload release button and an articulation yoke removed, a firing rod in a retracted position, and the lockout in the unlocked position;
FIG. 14 is a side perspective view from a second side of the central portion of the surgical stapling device shown in FIG. 10 with a rotation knob shown in phantom and the lockout assembly in the unlocked position;
FIG. 15 is a cross-sectional view taken along section line 15-15 of FIG. 14 with the lockout in the locked position;
FIG. 16 is a side perspective view of the first side of the central portion of the surgical stapling device shown in FIG. 1 with a rotation knob shown in phantom, the articulation yoke in a non-neutral position, and the lockout in a locked position;
FIG. 17 is a cross-sectional view taken along section line 17-17 of FIG. 16;
FIG. 18 is a cross-sectional view taken along section line 18-18 of FIG. 17;
FIG. 19 is a cross-sectional view taken along section line 19-19 of FIG. 10; and
FIG. 20 is a cross-sectional view taken through the central portion of the surgical stapling device shown in FIG. 10 with the firing rod in a partially advanced position and the lockout in a locked position.

### DETAILED DESCRIPTION

The disclosed surgical device will now be described in detail with reference to the drawings in which like reference numerals designate identical or corresponding elements in each of the several views. However, it is to be understood that the aspects of the disclosure are merely exemplary of the disclosure and may be embodied in various forms. Well-known functions or constructions are not described in detail to avoid obscuring the disclosure in unnecessary detail. Therefore, specific structural and functional details disclosed herein are not to be interpreted as limiting, but merely as a basis for the claims and as a representative basis for teaching one skilled in the art to variously employ the disclosure in virtually any appropriately detailed structure.

In this description, the term "proximal" is used generally to refer to that portion of the device that is closer to a clinician during use of the device in its customary fashion, while the term "distal" is used generally to refer to that portion of the device that is farther from the clinician during use of the device in its customary fashion. In addition, the term "clinician" is used generally to refer to medical personnel including doctors, nurses, and support personnel.

This disclosure is directed to a surgical stapling device that includes an adapter assembly having a lockout that prevents operation of a reload release mechanism of the adapter assembly when a firing rod of the adapter assembly is not in a fully retracted position and/or when an articulation mechanism of the adapter assembly is not in a neutral position.

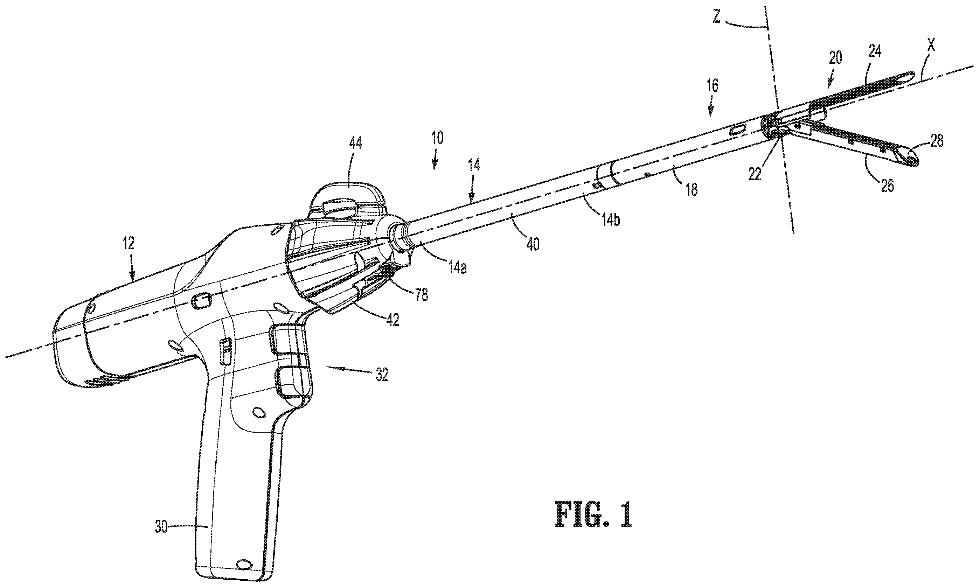

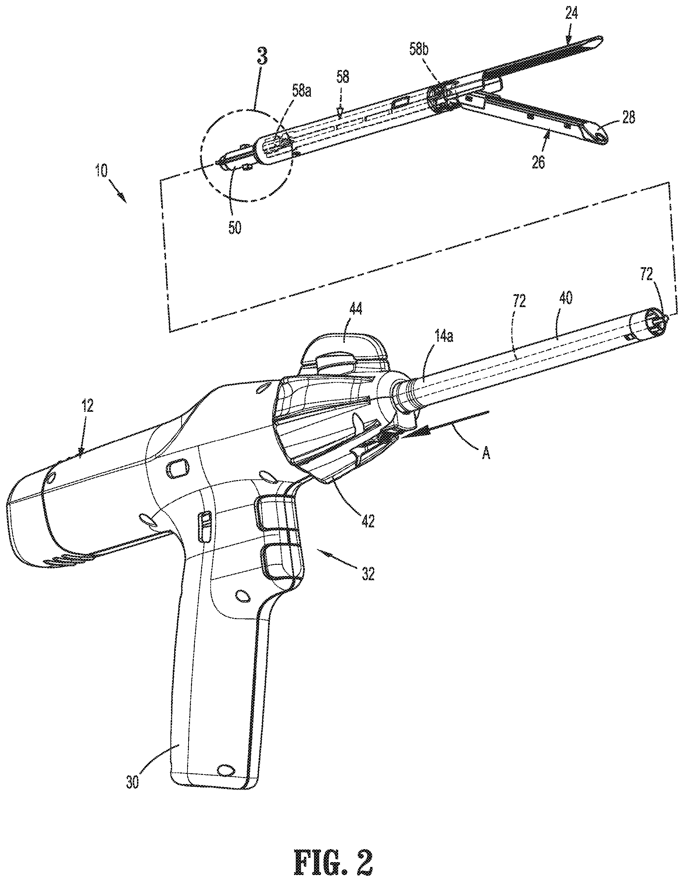

FIGS. 1 and 2 illustrate exemplary aspects of the disclosed surgical stapling device shown generally as stapling device 10. The stapling device 10 includes a powered handle assembly 12, an adapter assembly 14, and a reload assembly 16. The adapter assembly 14 defines a longitudinal axis "X" and includes a proximal portion 14a supported on the handle assembly 12 and a distal portion 14b that supports the reload assembly 16. The reload assembly 16 includes a proximal body 18 and a tool assembly 20. The tool assembly is pivotably coupled to a distal portion of the proximal body 18 by a pivot member 22 to facilitate articulation of the tool assembly 20 in relation to the proximal body 18 about an articulation axis "Z" that is transverse to the longitudinal axis "X" of the adapter assembly 14. The proximal body 18 of the reload assembly 16 has a proximal portion that is adapted to be releasably coupled to the distal portion 14b of the adapter assembly 14 of the stapling device 10.

In aspects of the disclosure, the tool assembly 20 includes an anvil 24 and a cartridge assembly 26. The cartridge assembly 26 includes a staple cartridge 28 that supports a plurality of staples (not shown). For a description of exemplary aspects of a tool assembly suitable for use with the stapling device 10, see, e.g., U.S. Patent No. 6,241,139 ("the '139 patent"). It is envisioned that aspects of this disclosure are suitable for use with other types of surgical devices having replaceable reload assemblies including clip appliers, ligation devices, or the like.

The handle assembly 12 of the stapling device 10 includes a stationary handle 30 and actuation buttons 32 that can be depressed to actuate the tool assembly 20, e.g., approximate the tool assembly 20, and fire staples from the staple cartridge 28. In aspects of the disclosure, batteries (not shown) are supported in the stationary handle 20 to power the handle assembly 12.

The adapter assembly 14 includes an outer tube 40 that supports a rotation knob 42. The rotation knob 42 is rotatably coupled to the handle assembly 12 and fixedly coupled to the proximal portion 14b of the adapter assembly 14 such that rotation of the rotation knob 42 in relation to the handle assembly 12 causes corresponding rotation of the adapter assembly 14 and the reload assembly 16 about the longitudinal axis "X". The rotation knob 42 supports an articulation assembly that includes an articulation lever 44 which is described in further detail below.

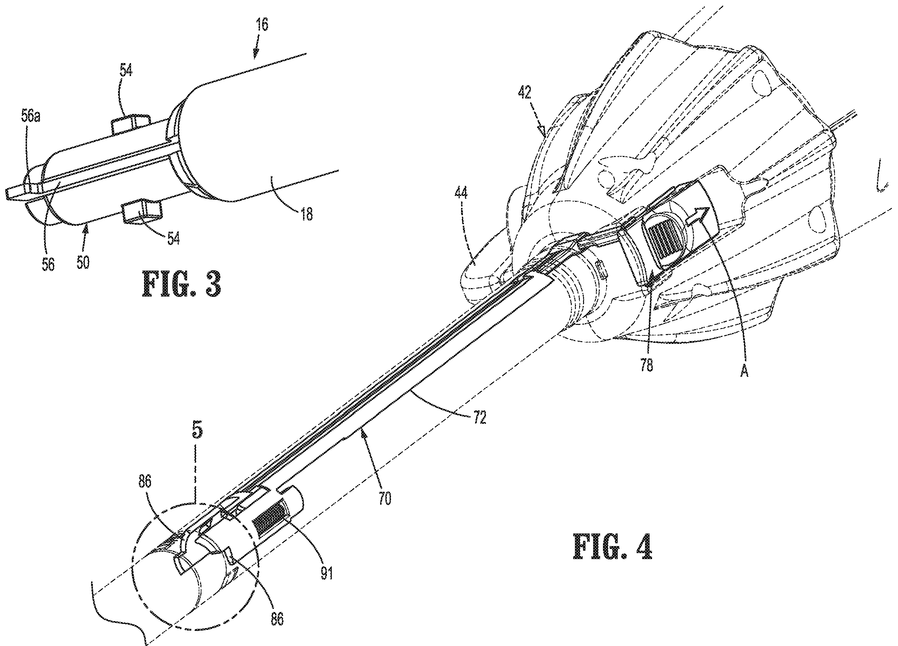

FIGS. 2-9 illustrate the connection between the proximal body 18 of the reload assembly 16 and the distal portion 14b of the adapter assembly 14. The proximal body 18 of the reload assembly 16 includes a connector 50 that includes a cylindrical body 52 and two diametrically opposed nubs 54 that project outwardly from the body 52. The proximal body 18 of the reload assembly 16 also includes an articulation link 56 (FIG. 3) and a drive assembly 58 (shown in phantom in FIG. 2). The articulation link 56 includes a proximal portion that includes a finger 56a that is configured to be coupled to the articulation assembly in the adapter assembly 14, as described in further detail below, when the reload assembly 16 is coupled to the adapter assembly 14. The articulation link 56 also includes a distal portion 56b (FIG. 2) that is coupled to the tool assembly 20 at a location offset from the articulation axis "Z" (FIG. 1) such that longitudinal movement of the articulation link 56 causes the tool assembly 20 to articulate about the articulation axis "Z".

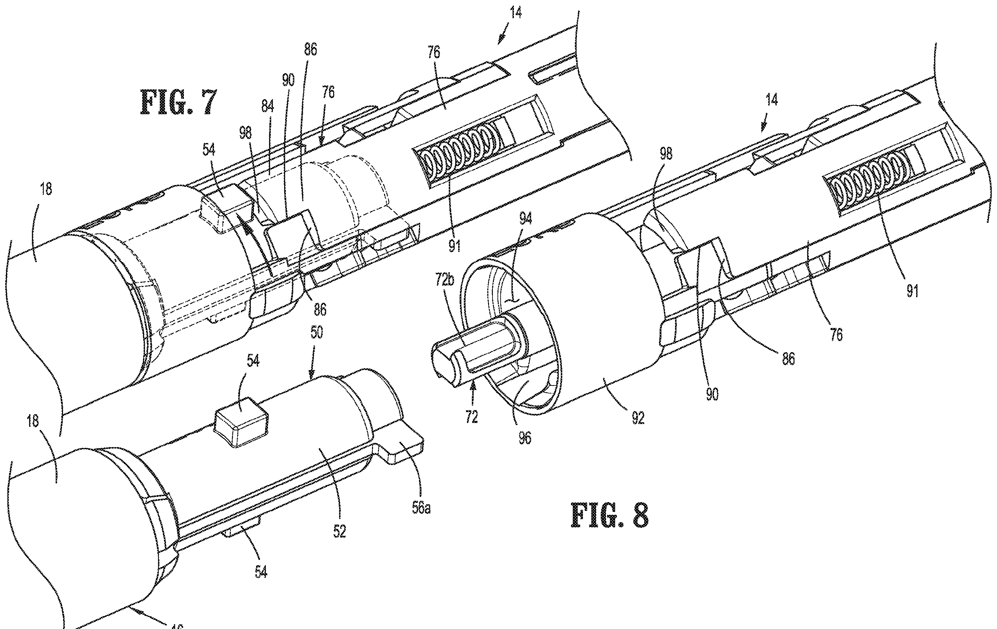

The drive assembly 58 includes a proximal portion 58a that is adapted to be coupled to a firing rod 72 (FIG. 8) of the adapter assembly 14 when the reload assembly 16 is coupled to the adapter assembly 14. A distal portion of the drive assembly 58 includes a working end 58b that is engaged with the anvil 24 and the cartridge assembly 26 and is movable along the length of the tool assembly 20 to eject staples (not shown) from the staple cartridge 28. In aspects of the disclosure, the working end 58b of the drive assembly 58 has an I-beam configuration and supports a cutting blade (not shown). U.S. Patent No. 8,286,848 discloses a surgical device with a reload assembly that is releasably coupled to an adapter assembly and includes the features described herein that is suitable for use in the stapling device 10.

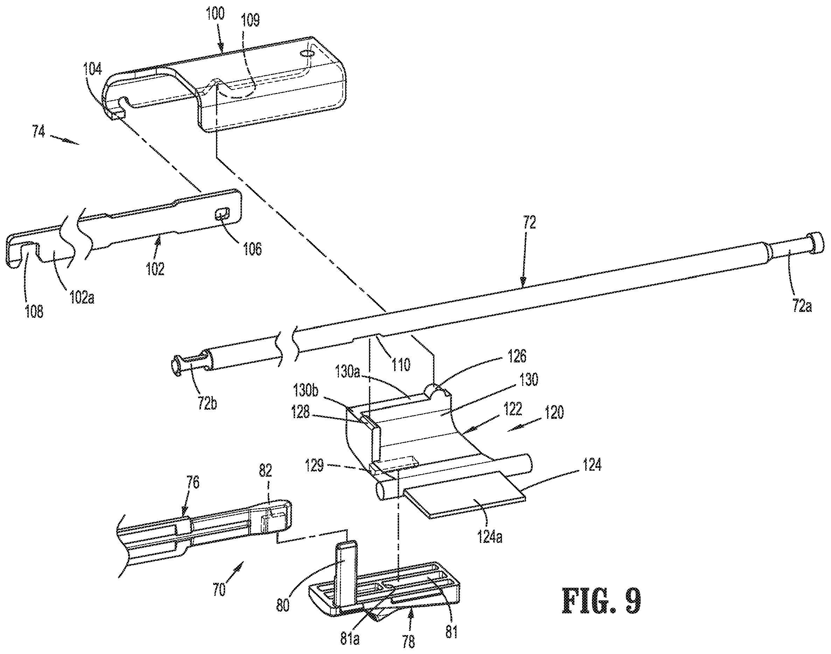

The adapter assembly 14 includes a reload release mechanism 70 (FIG. 9), a firing rod 72, and an articulation mechanism 74 (FIG. 9). The release mechanism 70 includes a release member 76 and a release button 78. The release button 78 is coupled to a proximal portion of the release member 76 and is supported on the rotation knob 42 for movement between advanced and retracted positions to move the release member 76 between locked and unlocked positions. In aspects of the disclosure, the release button 78 includes a finger 80 (FIG. 9) that is received within a blind bore 82 (FIG. 9) formed in the proximal portion of the release member 76. Alternately, other coupling techniques or devices can be used to couple the release button 78 to the release member 76. The release button 78 includes an inner surface that forms a cavity 81 that is defined in part by a stop surface 81a.

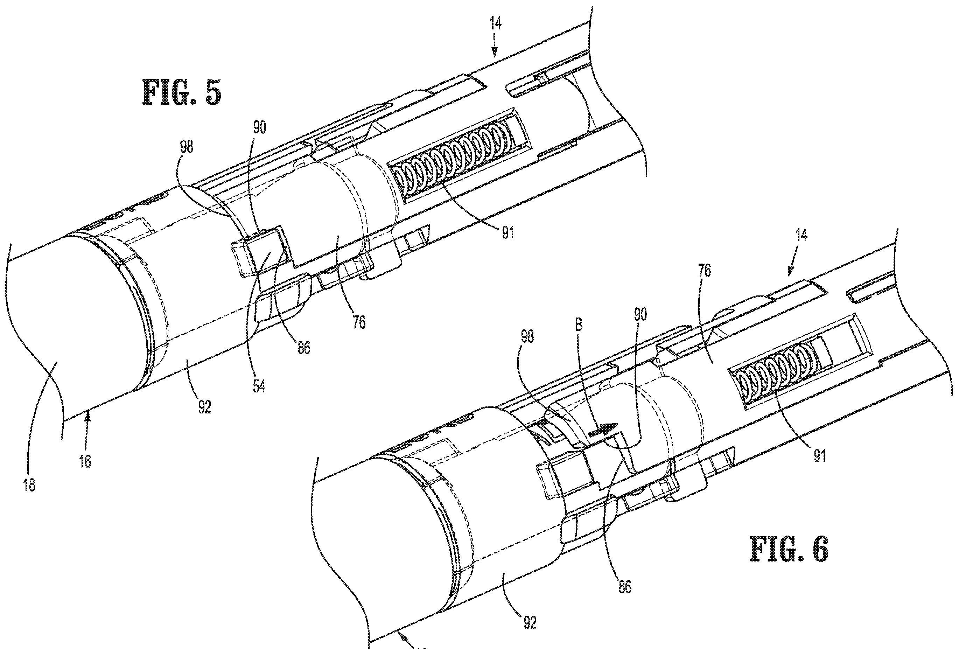

The release member 76 includes a distal portion 84 (FIG. 6) that defines cutouts 86 (FIG. 4) that receive the nubs 54 of the reload assembly 16 when the release member 76 is in the locked position (FIG. 5). The cutouts 86 are defined in part by side walls 90 (FIG. 6) that engage the nubs 54 of the reload assembly 16 when the release member 76 is in the locked position (FIG. 5) to prevent rotation of the reload assembly 16 in relation to the adapter assembly 14. A biasing member 91 is positioned within the outer tube 40 of the adapter assembly 14 to urge the release member 76 distally to the locked position.

The distal portion 14b of the adapter assembly 14 includes a connector 92 (FIG. 8) that defines a cylindrical bore 94. The connector 92 includes inner walls that define L-shaped channels 96 (FIG. 8) (only one is shown) that receive the nubs 54 on the connector 50 of the reload assembly 16 when the reload assembly 16 is inserted into the cylindrical bore 96. The L-shaped channels 96 are axially aligned with a distal end 98 of the release member 76. When the connector 50 of the reload assembly 16 is inserted into the connector 92 of the adapter assembly 14 and the nubs 54 are slid into the L-shaped channels 96 (FIG. 8), engagement between the nubs 54 and the distal end 98 of the release member 76 moves the release member 76 from its locked position towards its unlocked position compressing the biasing member 91. After the nubs 54 of the connector 50 of the reload assembly 16 are moved to a position aligned with a transverse portion (not shown) of the L-shaped channels 96, the reload assembly 16 is rotated in relation to the adapter assembly 14 to move the nubs 54 into the transverse portion of the L-shaped channels 96. When the nubs 54 of the connector 50 are rotated past the distal end 98 of the release member 76 to a position aligned with the cutouts 86 in the release member 76, the biasing member 91 urges the release member 76 back the locked position (FIG. 5). In the locked position of the release member 76, the side walls 90 of the release member 76 engage the nubs 54 of the reload assembly 16 to prevent the reload assembly 16 from being rotated in relation to the adapter assembly 14 and, thus, lock the reload assembly 16 onto the adapter assembly 14.

FIGS. 6-8 illustrate operation of the reload release mechanism 70. When the release button 78 is slid proximally in the direction of arrow "A" in FIG. 2, the release member 76 moves proximally in the direction of arrow "B" in FIG. 6 from the locked position (FIG. 5) to the unlocked position (FIG. 6). In the unlocked position, the side walls 90 defining the cutouts 86 in the distal portion 84 of the release member 76 are positioned proximally of the nubs 54 of the reload assembly 16 such that the reload assembly 16 can be rotated in relation to the adapter assembly 14 to disengage the reload assembly 16 from the adapter assembly 14 (FIG. 8).

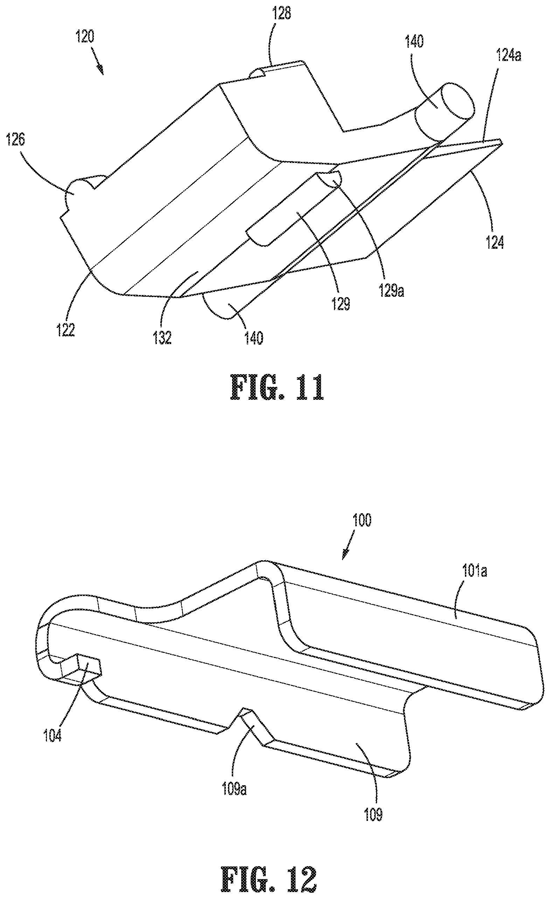

The articulation assembly 74 includes a coupling member 100 and an articulation link 102. The coupling member 74 is coupled to the articulation lever 44 (FIG. 1) of the articulation assembly by a mechanism (not shown) for translating pivotable movement of the articulation lever 44 into longitudinal movement of the coupling member 100. The coupling member 100 is secured to the articulation link 102 such that longitudinal movement of the coupling member 100 between a neutral position and non-neutral positions causes longitudinal movement of the articulation link 102 between advanced and retracted positions. In aspects of the disclosure, the coupling member 100 includes a finger 104 (FIG. 9) and the articulation link 102 has a proximal portion that defines an opening 106 that receives the finger 104 to secure the coupling member 100 to the articulation link 102. In aspects of the disclosure, the coupling member 100 has a U-shaped body 100a having a side wall 109 that defines a first notch 109a (FIG. 12).

The articulation link 102 includes a distal portion 102a that is adapted to be coupled to the articulation link 56 (FIG. 9) of the reload assembly 16. In aspects of the disclosure, the distal portion 102a of the articulation link 102 of the adapter assembly 14 defines a slot 108 that receives the proximal portion 56a of the articulation link 56 of the reload assembly 16 (FIG. 3). When the reload assembly 16 is rotatably coupled to the adapter assembly 14, the proximal portion 56a of the articulation link 56 is received within the slot 108 in the articulation link 102 to couple the articulation link 102 of the adapter assembly 14 to the articulation link 56 (FIG. 3) of the reload assembly 16. When the reload assembly 16 is coupled to the adapter assembly 14, longitudinal movement of the articulation link 102 causes longitudinal movement of the articulation link 56 of the reload assembly 16 to articulate the tool assembly 20 in relation to the proximal body 18 of the reload assembly 16 about the articulation axis "Z".

The firing rod 72 of the adapter assembly 14 includes a proximal portion 72a that is coupled to the handle assembly 12 such that actuation of the handle assembly 12 causes longitudinal movement of the firing rod 72. The firing rod 72 also includes a distal portion 72b (FIG. 8) that is coupled to the drive assembly 58 of the reload assembly 16 when the reload assembly 16 is coupled to the adapter assembly 14 of the stapling device 10 such that longitudinal movement of the firing rod 72 within the outer tube 40 of the adapter assembly 14 causes longitudinal movement of the drive assembly 58 (FIG. 2) within the reload assembly 16. The firing rod 72 defines a second notch 110.

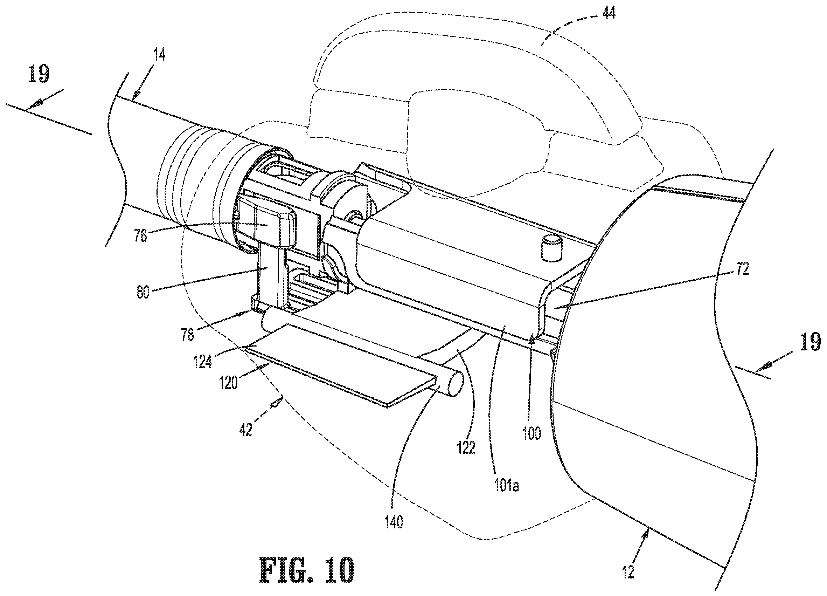

FIGS. 9-12 illustrate the lockout 120 which prevents movement of the release button 78 from its advanced position towards its retracted position unless the coupling member 100 of the articulation assembly of the adapter assembly 14 (FIG. 1) is in its neutral position and the firing rod of the adapter assembly 14 is in the retracted position. The lockout 120 includes a body 122 and a biasing member 124 that is coupled to the body 122. In aspects of the disclosure, the biasing member 124 is in the form of a leaf spring 124a that is secured to or integrally formed with the body 122 of the lockout 120. The body 122 of the lockout 120 includes a first protrusion 126 that is received within the first notch 109 (FIG. 12) of the coupling member 100 of the articulation assembly when the coupling member 100 is in its neutral position and a second protrusion 128 that is received within the second notch 110 (FIG. 9) of the firing rod 72 when the firing rod 72 is in the retracted position. The body 122 also includes a stop member 129 (FIG. 11) that is positioned on a bottom wall 132 of the body 122 and has a proximally facing stop surface 129a. The stop member 129 is positioned above the cavity 81 (FIG. 9) in the release button 78. In aspects of the disclosure, the body 122 of the lockout 120 includes an L-shaped wall 130 that extends upwardly (as viewed in FIG. 9) from the bottom wall 132 and has a longitudinal portion 130a and a transverse portion 130b. The first protrusion 126 is supported on top of the longitudinal portion 130a of the L-shaped wall 130 and the second protrusion 128 is positioned on top of the transverse portion 130b of the L-shaped wall 130.

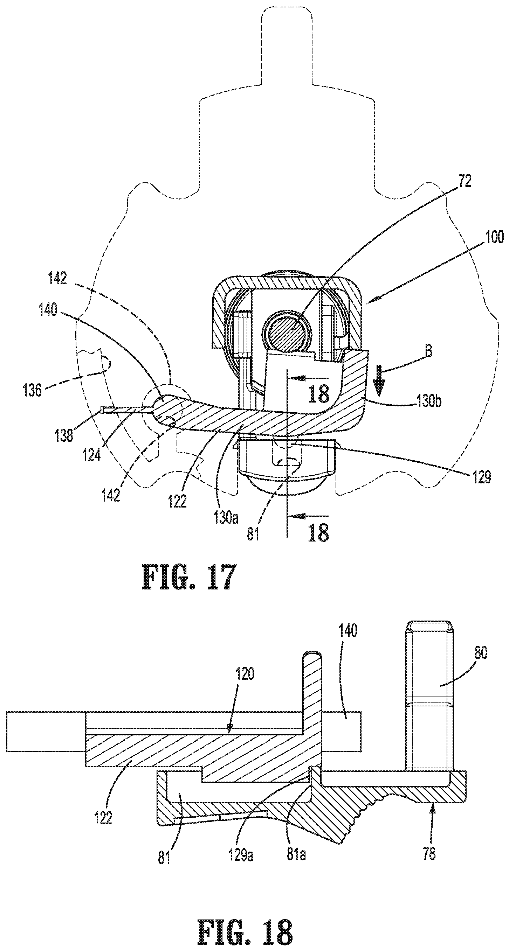

The biasing member 124 of the lockout 120 is secured to an inner wall 136 of the rotation knob 42. In aspects of the disclosure, the biasing member 124 is secured within a slot 138 in the inner wall 136 (FIG. 17) of the rotation knob 42 such that the body 122 of the lockout 120 is supported in cantilevered fashion within the rotation knob 42. In certain aspects of the disclosure, the body 122 of the lockout 120 includes a cylindrical pivot member 140 that is positioned between the biasing member 124 and the L-shaped wall 130 and is supported within a circular channel 142 (FIG. 17) of a support member 144. In aspects of the disclosure, the support member 144 is integrally formed with the rotation knob 42.

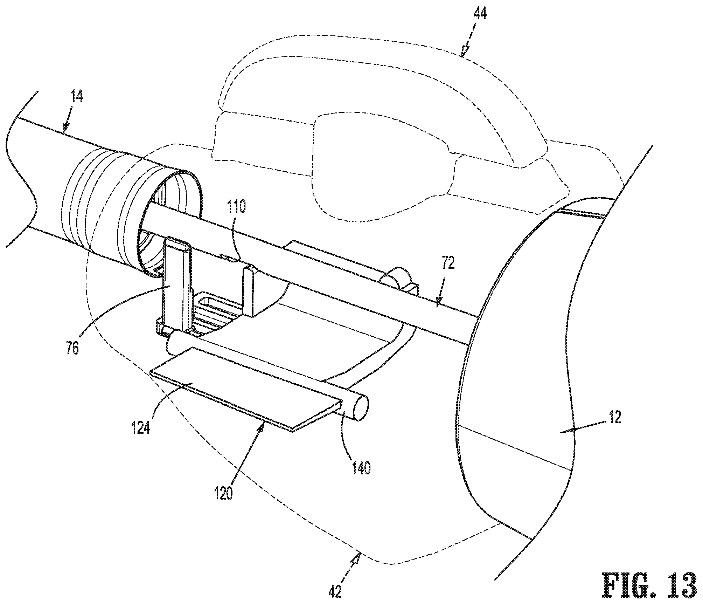

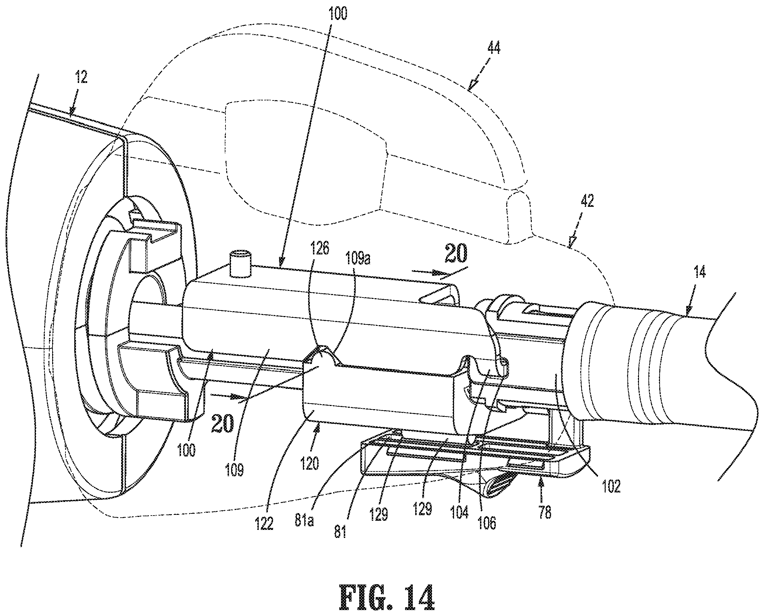

FIG. 13 illustrates the lockout 120 and the firing rod 72 of the adapter assembly 14 when the firing rod 72 is in its retracted position. When the firing rod 72 is in a retracted position, the second protrusion 128 of the lockout 120 is received within the second notch 110 of the firing rod 72 and the biasing member 124 of the lockout 120 is in a non-deformed position. In the non-deformed position, the stop member 129 is positioned above the cavity 81 (FIG. 14) of the release button 78 such that the stop surface 129a of the stop member 129 is not aligned with the stop surface 81a of the release button 78. In the non-deformed position, the release button 78 is movable from its advanced position to its retracted position to unlock the reload assembly 16 from the adapter assembly 14 and permit removal of the reload assembly 16 from the adapter assembly 14.

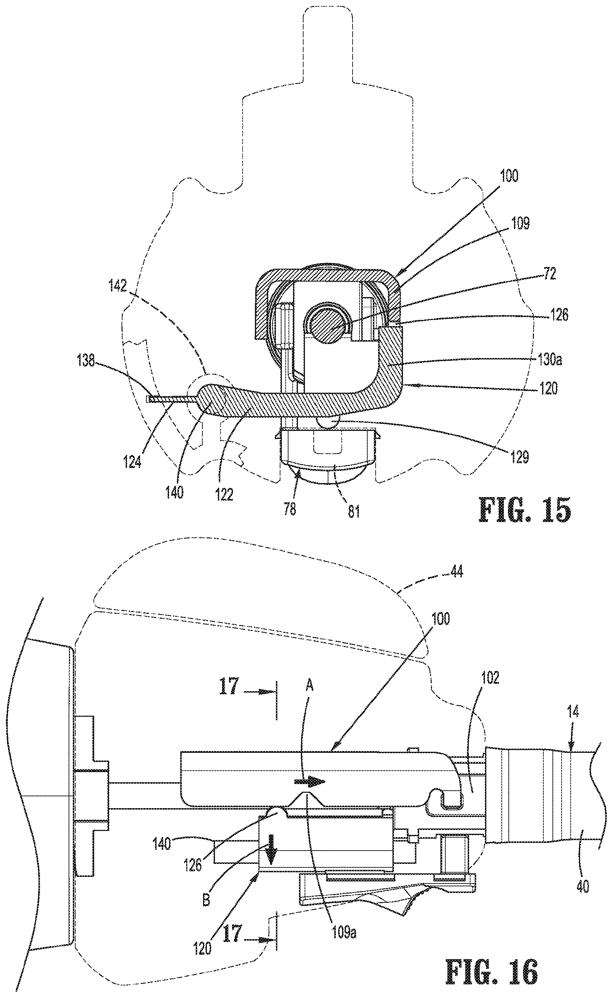

FIGS. 14 and 15 illustrate the lockout 120 and the coupling member 100 of the articulation assembly of the adapter assembly 14 with the coupling member 100 in the neutral position, i.e., the position in which a longitudinal axis of the tool assembly 20 is substantially coaxial with the longitudinal axis "X" of the adapter assembly 14. When the coupling member 100 is in the neutral position, the first protrusion 126 of the lockout 120 is received within the first notch 109a in the side wall 109 of the coupling member 100 and the biasing member 124 (FIG. 15) of the lockout 120 is in a non-deformed position. In the non-deformed position, the stop member 129 (FIG. 15) is positioned above the cavity 81 of the release button 78 such that the stop surface 129a of the stop member 129 is not aligned with the stop surface 81a of the release button 78. In the non-deformed position, the release button 78 is movable from its advanced position to its retracted position to unlock the reload assembly 16 and permit removal of the reload assembly 16 from the adapter assembly 14.

FIGS. 16-18 illustrate the lockout 120 as the coupling member 100 of the articulation assembly of the adapter assembly 14 moves to a non-neutral position. In the non-neutral position, the coupling member 100 has been moved longitudinally from the neutral position to move the articulation links 102 and 56 of the adapter assembly 14 and the reload assembly 20, respectively, longitudinally to pivot the tool assembly 20 about the articulation axis "Z". When the articulation lever 44 is rotated to advance (or retract) the coupling member 100 of the articulation assembly of the adapter assembly 14 in the direction indicated by arrow "A" in FIG. 16 from the neutral position to a non-neutral position, the first protrusion 126 on the body 122 of the lockout 120 moves from within the first notch 109a in the side wall 109 of the lockout 120 and engages the side wall 109 of the coupling member 100. Engagement of the first protrusion 126 and the side wall 109 of the coupling member 100 causes biasing member 124 to deform and the body 122 of the lockout 120 to pivot downwardly about the pivot member 140 in the direction of arrows "B" in FIGS. 16 and 17. When the body 122 of the lockout 120 moves downwardly in the direction of arrows "B", the stop member 129 on the lockout 120 moves into the cavity 81 in the release button 78 of the reload release mechanism 70. In this position, the stop surface 129a of the lockout 120 is aligned with the stop surface 81a on the release button 78 to prevent movement of the release button 78 from its advanced position towards its retracted position and prevent movement of the release member 76 from its locked position to its unlocked position. Thus, when the articulation mechanism of the adapter assembly 14 is in non-neutral position such that the tool assembly 20 is in an articulated position, the reload assembly 16 (FIG. 1) cannot be removed from the adapter assembly 14.

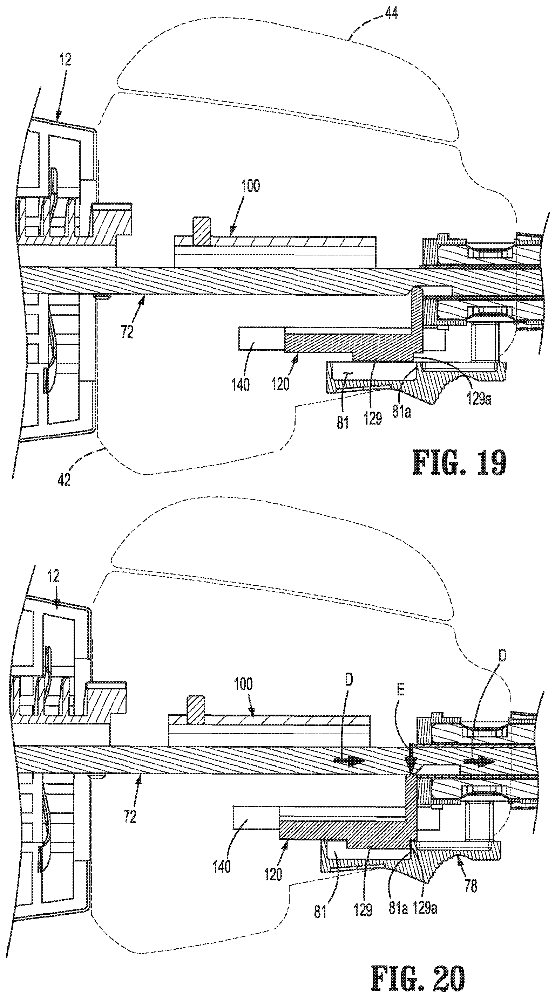

FIGS. 19 and 20 illustrate the lockout 120 and the firing rod 72 of the adapter assembly 14 when the firing rod 72 is moved in the direction of arrows "D" in FIG. 20 to a partially advanced position. When the firing rod 72 is in a partially advanced position, the second protrusion 128 of the lockout 120 is moved from the second notch 110 of the firing rod 72 and engages an outer surface of the firing rod 72. Engagement of the second protrusion 128 with the outer surface of the firing rod 72 causes biasing member 124 of the lockout 120 to deform such that the body 122 of the lockout 120 pivots downwardly about the pivot member 140 in the direction of arrows "E" in FIG. 20. When the body 122 of the lockout 120 moves downwardly in the direction of arrows "E", the stop member 129 on the lockout 120 moves into the cavity 81 in the release button 78 of the reload release mechanism 70. In this position, the stop surface 129a of the lockout 120 is aligned with the stop surface 81a on the release button 78 to prevent movement of the release button 78 from its advanced position towards its retracted position which prevents movement of the release member 76 from its locked position to its unlocked position. Thus, when the firing rod 72 of the adapter assembly 14 is in an advanced or partially advanced position, the reload assembly 16 (FIG. 1) cannot be removed from the adapter assembly 14.

The lockout 120 functions to prevent separation of the reload assembly 16 from the adapter assembly 14 of the stapling device 10 (FIG. 1) when the tool assembly 20 is in an articulated position and/or the firing rod 72 of the adapter assembly 14 is in an advanced or partially advanced position. If the tool assembly 20 is in an articulated position and/or the firing rod 72 is in a partially advanced or advanced position, the lockout 120 prevents operation of the reload release mechanism 70 to prevent separation of the reload assembly 16 from the adapter assembly 14 of the stapling device 10. Although the lockout is described in association with a firing rod and a coupling member of an articulation assembly, it is envisioned that the lockout 120 can be used in association with only a firing rod 72 or only a coupling member 100 of an articulation mechanism to lock or unlock the reload release mechanism 70.

Persons skilled in the art will understand that the devices and methods specifically described herein and illustrated in the accompanying drawings are nonlimiting exemplary aspects of the disclosure. It is envisioned that the elements and features illustrated or described in connection with one exemplary embodiment may be combined with the elements and features of another without departing from the scope of the present disclosure. As well, one skilled in the art will appreciate further features and advantages of the disclosure based on the above-described aspects of the disclosure. Accordingly, the disclosure is not to be limited by what has been particularly shown and described, except as indicated by the appended claims.

## Claims

1. An adapter assembly (14) comprising:
a firing rod (72) including a proximal portion and a distal portion, the firing rod (72) movable between retracted and advanced positions;
an articulation assembly (74) including an articulation lever (44), a coupling member (100), and an articulation link (102), the coupling member (100) positioned between the articulation lever (44) and the articulation link (102), the articulation lever (44) rotatable to move the coupling member (100) longitudinally between a neutral position and non-neutral positions to move the articulation link (102) between advanced and retracted positions;
a release mechanism (70) movable from a locked position to an unlocked position; and
a lockout (120) movable from an unlocked position to a locked position in response to movement of the coupling member (100) from the neutral position to one of the non-neutral positions and/or in response to movement of the firing rod (72) from the retracted position towards the advanced position, the lockout (120) configured to prevent movement of the release mechanism (70) from its locked position to its unlocked position when the lockout (120) is in its locked position wherein the lockout (120) includes a body (122) having a first protrusion (126) and a second protrusion (128), the coupling member (100) of the articulation assembly (74) defines a first notch (109), and the firing rod (72) defines a second notch (110), the first protrusion (126) positioned within the first notch (109) when the coupling member (100) is in the neutral position, and the second protrusion (128) positioned within the second notch (110) when the firing rod (72) is in the retracted position.

2. The adapter assembly of claim 1, wherein the body of the lockout (120) includes a stop member (129), and the release mechanism (70) includes a release button (78) that defines a cavity (81), the stop member of the body of the lockout (120) received within the cavity when the lockout (120) is in its locked position.

3. The adapter assembly of claim 2, wherein the lockout (120) includes a biasing member (91) that urges the lockout (120) towards its unlocked position.

4. The adapter assembly of claim 3, wherein the biasing member (91) includes a leaf spring.

5. The adapter assembly of claim 4, wherein the body of the lockout (120) is supported in cantilevered fashion from the leaf spring.

6. The adapter assembly of claim 5, wherein the body includes a cylindrical pivot member (140), the lockout (120) pivotable on the cylindrical pivot member (140) between the locked and unlocked positions.

7. The adapter assembly of claim 2, wherein the release mechanism (70) includes a release member (76) that is coupled to the release button.

8. A surgical stapling device (10) comprising:
a reload assembly (16) including a proximal body (18) and a tool assembly (20), the proximal body (18) defining a first longitudinal axis, the tool assembly (20) pivotably coupled to the proximal body (18) by a pivot member (22), the pivot member (22) defining an articulation axis (Z) that is transverse to the first longitudinal axis, the tool assembly (20) including a cartridge assembly (26) and an anvil (24), the proximal body (18) including a drive assembly (58) and a further articulation link (56), the further articulation link having a proximal portion and a distal portion, the distal portion of the further articulation link coupled to the tool assembly (20) such that movement of the further articulation link causes articulation of the tool assembly (20) about the articulation axis between a non-articulated position and articulated positions, the drive assembly (20) movable between retracted and advanced positions to actuate the tool assembly (20); and
the adapter assembly (14) according to any of claims 1 to 7, wherein the adapter assembly (14) defines a second longitudinal axis and having a distal portion (14b) releasably coupled to the reload assembly (16),
wherein the distal portion of the firing rod adapted to be coupled to the drive assembly of the reload assembly when the reload assembly is coupled to the adapter assembly, the firing rod movable between retracted and advanced positions to move the drive assembly of the reload assembly between its retracted and advanced positions;
wherein the articulation link of the articulation assembly is adapted to be coupled to the further articulation link of the proximal body of the reload assembly when the reload assembly is coupled to the adapter assembly such that longitudinal movement of the articulation link of the articulation assembly causes longitudinal movement of the further articulation link of the proximal body of the reload assembly to articulate the tool assembly about the articulation axis; and
wherein when the release mechanism (70) of the adapter assembly (14) is in the locked position, the release mechanism (70) prevents the reload assembly from being uncoupled from the adapter assembly.

9. The surgical stapling device (10) of claim 8, further including a handle assembly coupled to the adapter assembly (14).

10. The surgical stapling device (10) of claim 9, further including a rotation knob (42), the rotation knob rotatably coupled to the handle assembly and secured to the proximal portion of the adapter assembly (14), wherein rotation of the rotation knob in relation to the handle assembly rotates the adapter assembly (14) and the tool assembly (20) about the second longitudinal axis.

## Patentansprüche

1. Adapteranordnung (14), die Folgendes umfasst:
eine Auslösestange (72), die einen proximalen Abschnitt und einen distalen Abschnitt umfasst, wobei die Auslösestange (72) zwischen einer zurückgezogenen und einer vorgeschobenen Position bewegbar ist;
eine Gelenkanordnung (74), die einen Gelenkhebel (44), ein Kopplungselement (100) und ein Gelenkverbindungsglied (102) umfasst, wobei das Kopplungselement (100) zwischen dem Gelenkhebel (44) und dem Gelenkverbindungsglied (102) positioniert ist, wobei der Gelenkhebel (44) drehbar ist, um das Kopplungselement (100) in Längsrichtung zwischen einer neutralen Position und einer nicht neutralen Position zu bewegen, um das Gelenkverbindungsglied (102) zwischen einer vorgeschobenen und einer zurückgezogenen Position zu bewegen;
einen Freigabemechanismus (70), der aus einer verriegelten Position in eine entriegelte Position bewegbar ist; und
eine Verriegelung (120), die als Reaktion auf eine Bewegung des Kopplungselements (100) aus der neutralen Position in eine der nicht-neutralen Positionen und/oder als Reaktion auf eine Bewegung der Auslösestange (72) aus der zurückgezogenen Position in die vorgeschobene Position von einer entriegelten Position in eine verriegelte Position bewegbar ist, wobei die Verriegelung (120) dazu ausgelegt ist, eine Bewegung des Freigabemechanismus (70) aus seiner verriegelten Position in seine entriegelte Position zu verhindern, wenn sich die Verriegelung (120) in ihrer verriegelten Position befindet, wobei die Verriegelung (120) einen Körper (122) mit einem ersten Vorsprung (126) und einem zweiten Vorsprung (128) beinhaltet, das Kopplungselement (100) der Gelenkanordnung (74) eine erste Kerbe (109) definiert, und die Auslösestange (72) eine zweite Kerbe (110) definiert, wobei der erste Vorsprung (126) innerhalb der ersten Kerbe (109) positioniert ist, wenn sich das Kopplungselement (100) in der neutralen Position befindet, und der zweite Vorsprung (128) innerhalb der zweiten Kerbe (110) positioniert ist, wenn sich die Auslösestange (72) in der zurückgezogenen Position befindet.

2. Adapteranordnung nach Anspruch 1, wobei der Körper der Verriegelung (120) ein Anschlagelement (129) beinhaltet und der Freigabemechanismus (70) einen Freigabeknopf (78) beinhaltet, der einen Hohlraum (81) definiert, wobei das Anschlagelement des Körpers der Verriegelung (120) innerhalb des Hohlraums aufgenommen wird, wenn sich die Verriegelung (120) in ihrer verriegelten Position befindet.

3. Adapteranordnung nach Anspruch 2, wobei die Verriegelung (120) ein Vorspannelement (91) beinhaltet, das die Verriegelung (120) in Richtung ihrer entriegelten Position vorspannt.

4. Adapteranordnung nach Anspruch 3, wobei das Vorspannelement (91) eine Blattfeder umfasst.

5. Adapteranordnung nach Anspruch 4, wobei der Körper der Verriegelung (120) freitragend von der Blattfeder getragen ist.

6. Adapteranordnung nach Anspruch 5, wobei der Körper ein zylindrisches Schwenkelement (140) beinhaltet, wobei die Verriegelung (120) auf dem zylindrischen Schwenkelement (140) zwischen der verriegelten und der entriegelten Position schwenkbar ist.

7. Adapteranordnung nach Anspruch 2, wobei der Freigabemechanismus (70) ein Freigabeelement (76) beinhaltet, das mit dem Freigabeknopf gekoppelt ist.

8. Chirurgische Klammervorrichtung (10), die Folgendes umfasst:
eine Nachladeanordnung (16), die einen proximalen Körper (18) und eine Werkzeuganordnung (20) beinhaltet, wobei der proximale Körper (18) eine erste Längsachse definiert, wobei die Werkzeuganordnung (20) durch ein Schwenkelement (22) schwenkbar mit dem proximalen Körper (18) gekoppelt ist, wobei das Schwenkelement (22) eine Gelenkachse (Z) definiert, die quer zu der ersten Längsachse ist, wobei die Werkzeuganordnung (20) eine Kartuschenanordnung (26) und einen Amboss (24) beinhaltet, wobei der proximale Körper (18) eine Antriebsanordnung (58) und ein weiteres Gelenkverbindungsglied (56) beinhaltet, wobei das weitere Gelenkverbindungsglied einen proximalen Abschnitt und einen distalen Abschnitt aufweist, wobei der distale Abschnitt des weiteren Gelenkverbindungsglieds mit der Werkzeuganordnung (20) gekoppelt ist, so dass eine Bewegung des weiteren Gelenkverbindungsglieds eine Anlenkung der Werkzeuganordnung (20) um die Gelenkachse zwischen einer nicht angelenkten Position und angelenkten Positionen bewirkt, wobei die Antriebsanordnung (20) zwischen einer zurückgezogenen und einer vorgeschobenen Position bewegbar ist, um die Werkzeuganordnung (20) zu betätigen; und
Adapteranordnung (14) nach einem der Ansprüche 1 bis 7, wobei die Adapteranordnung (14) eine zweite Längsachse definiert und einen distalen Abschnitt (14b) aufweist, der lösbar mit der Nachladeanordnung (16) gekoppelt ist, wobei der distale Abschnitt der Auslösestange dazu ausgelegt ist, mit der Antriebsanordnung der Nachladeanordnung gekoppelt zu werden, wenn die Nachladeanordnung mit der Adapteranordnung gekoppelt ist, wobei die Auslösestange zwischen einer zurückgezogenen und einer vorgeschobenen Position beweglich ist, um die Antriebsanordnung der Nachladeanordnung zwischen ihrer zurückgezogenen und ihrer vorgeschobenen Position zu bewegen;
wobei das Gelenkverbindungsglied der Gelenkanordnung dazu ausgelegt ist, mit dem weiteren Gelenkverbindungsglied des proximalen Körpers der Nachladeanordnung gekoppelt zu werden, wenn die Nachladeanordnung mit der Adapteranordnung gekoppelt ist, so dass eine Längsbewegung des Gelenkverbindungsglieds der Gelenkanordnung eine Längsbewegung des weiteren Gelenkverbindungsglieds des proximalen Körpers der Nachladeanordnung bewirkt, um die Werkzeuganordnung um die Gelenkachse anzulenken; und
wobei, wenn sich der Freigabemechanismus (70) der Adapteranordnung (14) in der verriegelten Position befindet, der Freigabemechanismus (70) verhindert, dass die Nachladeanordnung von der Adapteranordnung entkoppelt wird.

9. Chirurgische Klammervorrichtung (10) nach Anspruch 8, ferner umfassend eine Griffanordnung, die mit der Adapteranordnung (14) gekoppelt ist.

10. Chirurgische Klammervorrichtung (10) nach Anspruch 9, ferner umfassend einen Drehknopf (42), wobei der Drehknopf drehbar mit der Griffanordnung gekoppelt und an dem proximalen Abschnitt der Adapteranordnung (14) befestigt ist, wobei eine Drehung des Drehknopfs in Bezug auf die Griffanordnung die Adapteranordnung (14) und die Werkzeuganordnung (20) um die zweite Längsachse dreht.

## Revendications

1. Ensemble adaptateur (14) comprenant :
une tige de déclenchement (72) comprenant une partie proximale et une partie distale, la tige de déclenchement (72) étant mobile entre des positions rétractée et avancée ;
un ensemble d'articulation (74) comprenant un levier d'articulation (44), un élément d'accouplement (100), et une liaison d'articulation (102), l'élément d'accouplement (100) étant positionné entre le levier d'articulation (44) et la liaison d'articulation (102), le levier d'articulation (44) étant mobile en rotation pour déplacer longitudinalement l'élément d'accouplement (100) entre une position neutre et des positions non neutres pour déplacer la liaison d'articulation (102) entre des positions avancée et rétractée ;
un mécanisme de libération (70) mobile d'une position verrouillée à une position déverrouillée ; et
un verrouillage (120) mobile d'une position déverrouillée à une position verrouillée en réponse au déplacement de l'élément d'accouplement (100) de la position neutre à l'une des positions non neutres et/ou en réponse au déplacement de la tige de déclenchement (72) de la position rétractée vers la position avancée, le verrouillage (120) étant configuré pour empêcher le déplacement du mécanisme de libération (70) de sa position verrouillée à sa position déverrouillée lorsque le verrouillage (120) est dans sa position verrouillée, le verrouillage (120) comprenant un corps (122) ayant une première saillie (126) et une seconde saillie (128), l'élément d'accouplement (100) de l'ensemble d'articulation (74) définissant une première encoche (109), et la tige de déclenchement (72) définissant une seconde encoche (110), la première saillie (126) étant positionnée à l'intérieur de la première encoche (109) lorsque l'élément d'accouplement (100) est en position neutre, et la seconde saillie (128) étant positionnée à l'intérieur de la seconde encoche (110) lorsque la tige de déclenchement (72) est en position rétractée.

2. Ensemble adaptateur selon la revendication 1, le corps du verrouillage (120) comprenant un élément d'arrêt (129), et le mécanisme de libération (70) comprenant un bouton de libération (78) qui définit une cavité (81), l'élément d'arrêt du corps du verrouillage (120) étant reçu à l'intérieur de la cavité lorsque le verrouillage (120) est dans sa position verrouillée.

3. Ensemble adaptateur selon la revendication 2, le verrouillage (120) comprenant un élément de sollicitation (91) qui pousse le verrouillage (120) vers sa position déverrouillée.

4. Ensemble adaptateur selon la revendication 3, l'élément de sollicitation (91) comprenant un ressort à lame.

5. Ensemble adaptateur selon la revendication 4, le corps du verrouillage (120) étant supporté en porte-à-faux par le ressort à lame.

6. Ensemble adaptateur selon la revendication 5, le corps comprenant un élément de pivot cylindrique (140), le verrouillage (120) pouvant pivoter sur l'élément de pivot cylindrique (140) entre les positions verrouillée et déverrouillée.

7. Ensemble adaptateur selon la revendication 2, le mécanisme de libération (70) comprenant un élément de libération (76) qui est couplé au bouton de libération.

8. Dispositif d'agrafage chirurgical (10) comprenant :
un ensemble de rechargement (16) comprenant un corps proximal (18) et un ensemble outil (20), le corps proximal (18) définissant un premier axe longitudinal, l'ensemble outil (20) étant couplé de manière pivotante au corps proximal (18) par un élément de pivot (22), l'élément de pivot (22) définissant un axe d'articulation (Z) transversal au premier axe longitudinal, l'ensemble outil (20) comprenant un ensemble cartouche (26) et une enclume (24), le corps proximal (18) comprenant un ensemble d'entraînement (58) et une autre liaison d'articulation (56), l'autre liaison d'articulation ayant une partie proximale et une partie distale, la partie distale de l'autre liaison d'articulation étant couplée à l'ensemble outil (20) de telle sorte que le déplacement de l'autre liaison d'articulation provoque l'articulation de l'ensemble outil (20) autour de l'axe d'articulation entre une position non articulée et des positions articulées, l'ensemble d'entraînement (20) étant mobile entre des positions rétractée et avancée pour actionner l'ensemble outil (20) ; et
l'ensemble adaptateur (14) selon l'une quelconque des revendications 1 à 7, l'ensemble adaptateur (14) définissant un second axe longitudinal et ayant une partie distale (14b) couplée de manière amovible à l'ensemble de rechargement (16),
la partie distale de la tige de déclenchement étant adaptée pour être couplée à l'ensemble d'entraînement de l'ensemble de rechargement lorsque l'ensemble de rechargement est couplé à l'ensemble adaptateur, la tige de déclenchement étant mobile entre des positions rétractée et avancée pour déplacer l'ensemble d'entraînement de l'ensemble de rechargement entre ses positions rétractée et avancée ;
la liaison d'articulation de l'ensemble d'articulation étant adaptée pour être couplée à l'autre liaison d'articulation du corps proximal de l'ensemble de rechargement lorsque l'ensemble de rechargement est couplé à l'ensemble adaptateur, de telle sorte qu'un déplacement longitudinal de la liaison d'articulation de l'ensemble d'articulation entraîne un déplacement longitudinal de l'autre liaison d'articulation du corps proximal de l'ensemble de rechargement pour articuler l'ensemble outil autour de l'axe d'articulation ; et
lorsque le mécanisme de libération (70) de l'ensemble adaptateur (14) est dans la position verrouillée, le mécanisme de libération (70) empêchant l'ensemble de rechargement d'être découplé de l'ensemble adaptateur.

9. Dispositif d'agrafage chirurgical (10) selon la revendication 8, comprenant en outre un ensemble de poignée couplé à l'ensemble adaptateur (14).

10. Dispositif d'agrafage chirurgical (10) selon la revendication 9, comprenant en outre un bouton de rotation (42), le bouton de rotation étant couplé de manière rotative à l'ensemble de poignée et fixé à la partie proximale de l'ensemble adaptateur (14), la rotation du bouton de rotation par rapport à l'ensemble de poignée faisant tourner l'ensemble adaptateur (14) et l'ensemble outil (20) autour du second axe longitudinal.
